# EUROPEAN PATENT APPLICATION

(11) **EP 2 756 827 A1**
(43) Date of publication of application: **23.07.2014**
(21) Application number: 13380002.9
(22) Date of filing: 21.01.2013
(51) Int. Cl.: A61F 9/00

(54) **Aid device for eye drops applicator**

(71) Applicant: De Miguel Simo, Pedro Victor, 08850 Gava Barcelona (ES); Huertas LLort, David, 08902 L'Hospitalet de Llobregat Barcelona (ES); Sala Serra, Alexandre, 08850 Gava Barcelona (ES)
(72) Inventor: De Miguel Simo, Pedro Victor, 08850 Gavà (Barcelona) (ES)
(74) Representative: Juncosa Miro, Jaime

(57) **Abstract**

The eye drop applicator device comprises a housing (H) defming an open shell having a perimetral edge adapted to the facial area surrounding a user's eye socket; a first opening (A1) for a nozzle of a first eye drop dispenser providing a first droplet fall trajectory towards the user's eye, a second opening (A2) for a nozzle of a second dispenser providing a second droplet fall trajectory towards the eye, and a sight orifice (A3) formed in the housing (H) and located between said first and second openings (A1, A2) in a position such that a sight line of the user's eye through the sight orifice (A3) does not cross either of said first and second droplet fall trajectories or their prolongations.

## Description

### Technical Field

The present invention generally relates to an eye drop applicator device which can be used with different eye drop dispenser types, and more particularly to an applicator device with several inlet openings, each of them provided for one of said different dispenser types.

### State of the Prior Art

Patent US 3446209 describes a device to facilitate eye drop application comprising a support structure configured for being placed on a patient's face in front of his/her eyes, held on the nose and ears in a manner similar to conventional spectacle frames, and an opening associated with a corresponding coupling element in front of each eye for coupling a nozzle of a droplet dispenser to be used on either of the two eyes.

Document WO 2007/068987 discloses an eye drop applicator comprising a housing with an edge configured for being applied around a patient's eye, an inlet opening in said housing for dispensing droplets, and a movable adaptor to adapt the applicator to the right or left eye.

Patent US 4531944 discloses an eye drop applicator which can be used with different dispenser types. The applicator is formed by a housing including a single inlet opening for dispensing droplets and having various external auxiliary parts which can alternatively be coupled to the inlet opening in order to enable using both dispenser types alternatively through the same inlet opening, each of said external auxiliary parts configured for coupling a particular nozzle of the dispenser type to be used.

The need of using such external auxiliary parts in the mentioned patent US 4531944 is obviously a clear drawback in terms of using the proposed applicator for different dispenser types because the user must remove an auxiliary part and put another auxiliary part in place when he/she wants or needs to use another dispenser type. Likewise, since these auxiliary parts are relatively small, there is a likelihood of losing them, whereby in such case the applicator could not be used with the dispenser that fits in the lost auxiliary part.

The mentioned patent US 4531944 also describes a sight opening in the form of an aperture formed in the housing in a position adjacent to the only inlet opening for dispensing droplets in order to distract the patient's eye directing his/her vision to a swiveling element moving in said aperture, provided by the sight opening and thereby to achieve that the patient successfully positions the dispenser so that the droplet falls vertically on the eye preventing the patient from closing his/her eye when applying the droplet. Nevertheless, in this applicator the sight line through the sight opening and the droplet fall trajectory towards the eye are in one and the same plane, i.e., the sight line and the prolongation of the droplet fall trajectory cross one another, and this has the drawback of causing the patient to close his/her eye due to reflex reaction when he/she sees that the droplet is falling.

### Disclosure of the Invention

It is necessary to offer an alternative to the state of the art which covers the drawbacks found therein and which particularly offers an eye drop applicator device which can be used for different dispenser types not having the drawbacks of the applicator of the mentioned patent US 4531944.

The present invention provides an eye drop applicator device comprising, as is already known, a housing provided with walls defining an open-bottom shell, and having a lower perimetral edge configured for being adapted to the facial area surrounding a user's eye socket, an inlet opening formed through one of said walls of the housing for inserting at least part of a nozzle of an eye drop dispenser, and a sight orifice formed through one of the walls of the housing in a position adjacent to said inlet opening.

The eye drop applicator device of the present invention is **characterized in that** said first inlet opening is associated with a first coupling element configured for coupling a nozzle of a first dispenser type, and in that the housing comprises a second inlet opening formed through one of the walls of the housing in a position adjacent to said sight orifice, said second inlet opening being associated with a second coupling element configured for coupling a nozzle of a second dispenser type.

Another essential feature of the eye drop applicator of the present invention is that the first and second coupling elements and their respective first and second inlet openings are provided so that the eye drop dispensed therethrough follows corresponding first and second droplet fall trajectories towards the user's eye, and the applicator device comprises a sight orifice formed through one of the walls of the housing and located between the first and second inlet openings in a position such that the sight line of the user's eye on which the housing is arranged through said sight orifice does not cross any of said first and second droplet fall trajectories or their prolongations.

With this arrangement, when the user views through said sight orifice he/she does not see the eye drop droplet which is falling along either of said first and second droplet fall trajectories, so the user does not try to blink as a reflex reaction, allowing the correct administration of the eye drop in his/her eye.

According to one embodiment, the first coupling element comprises, for example, an inner screw thread defined in part of the inner contour of a first tubular portion communicating with the first inlet opening or including same as part of the inner opening thereof. This inner screw thread is conjugated with a corresponding outer screw thread associated with the nozzle of the first dispenser type provided for coupling thereof.

In the same or in another embodiment, the second coupling element of the applicator device comprises an inner conical seat communicating with the second inlet opening or including same as part of the inner opening thereof. Said inner conical seat has a section increasing from the second inlet opening to the outside of the housing and is conjugated with an outer conical surface associated with the nozzle of the second dispenser type provided for coupling thereof.

According to one variant of this embodiment, the conical seat is defined in at least part of an inner contour of a second tubular portion projecting outwardly and/or inwardly with respect to the housing from at least one of the walls thereof.

Optionally, the second coupling element further comprises two grooves aligned with one another defined in opposite locations of the inner conical seat, provided for inserting the longitudinal edges of the flattened body of a single-dose dispenser.

The first coupling element formed by the inner screw thread and the corresponding first tubular portion is defined around a first central geometric axis aligned with the first inlet opening, and the second coupling element formed by the conical seat and the corresponding second tubular portion is defined around a second central geometric axis aligned with the second inlet opening. These first and second central geometric axes are preferably substantially parallel to one another. The sight orifice is coaxial with a third central axis forming an angle between 30 and 45 degrees with respect to said first and second central geometric axes. When the housing is arranged on the facial area surrounding the user's eye socket and the user's head is in a tilted-back position, each of the first and second central geometric axes of the first and second coupling elements is in a substantially vertical position.

In one embodiment, the side walls of the housing have a lower perimetral edge, a closing portion of which adopts a curvature suitable for fitting with a portion of the user's face adjacent to the eye.

According to another embodiment, an occlusion portion of the mentioned lower perimetral edge of the side walls of the housing adopts a prominent curvature suitable for acting as an occluder which applies pressure on and occludes the lacrimal sac of the user's eye when in use.

According to one embodiment, the first inlet opening is defined partially in the top wall of the housing and partially in the side wall which is adjacent to the upper eyelid of the user's eye in a position of use.

According to one embodiment, the second inlet opening is defined in the top wall of the housing. Both first and second central geometric axes of the first and second inlet openings have a certain inclination with respect to a plane forming at least the region of the top wall surrounding the second opening.

For one embodiment, the applicator device is configured for being used in relation to the user's left eye, and for another embodiment the applicator device is configured symmetrically for being used in relation to the user's right eye.

According to a preferred embodiment, the housing is made up of a one-piece body made for example of a plastic material integrally incorporating both first and second coupling elements.

According to another embodiment, the applicator device proposed by the present invention comprises more than two coupling elements corresponding to as many inlet openings.

### Brief Description of the Drawings

The foregoing and other advantages and features will be better understood from the following detailed description of exemplary embodiments with reference to the attached drawings which must be interpreted in an illustrative and non-limiting manner, in which:
Figure 1 is a front perspective view of an eye drop applicator device according to an embodiment of the present invention;
Figure 2 is a rear perspective view of the eye drop applicator device of Figure 1; and
Figure 3 is a rear plan view of the eye drop applicator.

### Detailed Description of Exemplary Embodiments

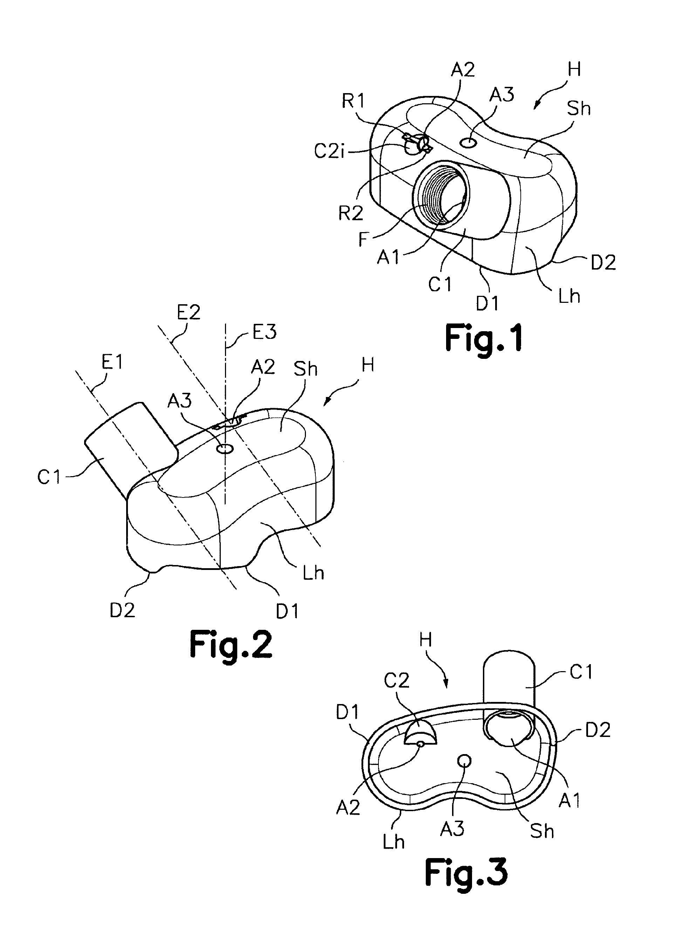

Figures 1, 2 and 3 show an embodiment of the eye drop applicator device of the present invention which comprises a housing H defining an open-bottom shell and is made up of a one-piece body made, for example, of a plastic material. Said housing H has a top wall Sh and side walls Lh connected to said top wall Sh by a rounded transition zone. The side walls Lh have a lower perimetral edge configured for being adapted to the facial area surrounding a user's eye socket.

The housing H has first and second inlet openings A1, A2 traversing the mentioned rounded transition zone between the top wall Sh and the side walls Lh of the housing H. For example, one part of the first inlet opening A1 traverses the top wall Sh of the housing H and another part of the first inlet opening A1 traverses one of the side walls Lh of the housing H.

In a position of use, the first and second inlet openings A1, A2 are adjacent to the upper eyelid of the user's eye. The applicator device includes first and second coupling elements associated with the first and second inlet openings A1, A2, respectively.

Said first coupling element serves for coupling and inserting a nozzle of a first eye drop dispenser type, such as a multi-dose dispenser (not illustrated), and said second coupling element serves for coupling a nozzle of a second eye drop dispenser type, such as a single-dose eye drop dispenser (not illustrated).

The first coupling element comprises an inner screw thread F formed in an inner surface of a first tubular portion C1 communicating with the first inlet opening A1 or including same as part of the inner opening thereof. The mentioned inner screw thread is conjugated with a corresponding outer screw thread associated with the nozzle of the first eye drop dispenser type provided for coupling thereof.

The second coupling element comprises a conical seat C2i formed in a second tubular portion C2, which, in the illustrated embodiment (Figure 3), projects inwardly with respect to the housing H. The mentioned conical seat C2i has a section increasing from the second inlet opening A2 to the outside of the housing H and is conjugated with an outer conical surface of the nozzle of the second eye drop dispenser type provided for coupling thereof.

The second coupling element further comprises two grooves R1, R2 aligned with one another formed on diametrically opposite sides of the conical seat C2i. These two grooves R1, R2 of the second coupling element are configured for receiving the longitudinal edges of a flattened body of a single-dose dispenser.

As can be seen in the attached drawings, the applicator device of the invention further comprises a sight orifice A3 traversing the top wall Sh of the housing H in a location such that the sight line of the user's eye through said sight orifice A3 does not cross any of the droplet fall trajectories towards the user's eye which the eye drop droplets dispensed through the first and second inlet openings A1 and A2 will follow.

The first coupling element formed by the inner screw thread F and the corresponding first tubular portion C1 is defined around a first central geometric axis E1 aligned with the first inlet opening A1, and the second coupling element formed by the conical seat C2i and the second tubular portion is defined around a second central geometric axis E2 aligned with the second inlet opening. In the embodiment shown, the first and second central geometric axes E1, E2 are parallel to one another.

The sight orifice A3 is centered in relation to a third central geometric axis E3 perpendicular to a plane formed by at least one region of the top wall Sh surrounding the sight orifice A3, and the first and second central geometric axes E1, E2 have a certain inclination with respect to the mentioned plane formed by the top wall Sh, and accordingly with respect to the third central geometric axis E3. For example, the third central geometric axis E3 forms an angle between 30 and 45 degrees with respect to the first and second central geometric axes E1, E2.

When the housing H is arranged in a position of use on the facial area surrounding the user's eye socket and the user's head is in a tilted-back position, the first and second central geometric axes E1, E2 of the first and second coupling elements are in a substantially vertical position, such that the first and second droplet fall trajectories towards the user's eye are aligned substantially with the first and second central geometric axes E1, E2.

As better shown in Figures 1 and 2, the lower perimetral edge of the side walls Lh of the housing H has a closing portion D 1 which adopts a curvature suitable for fitting with a portion of the user's face adjacent to the eye, and an occlusion portion D2 which adopts a curvature suitable for acting as an occluder which applies pressure on the lacrimal sac of the user's eye when in use.

## Claims

1. An eye drop applicator device of the type comprising:
- a housing (H) having walls defining an open-bottom shell and having a lower perimetral edge configured for being adapted to the facial area surrounding a user's eye socket;
- an inlet opening (A1) formed through one of said walls of the housing (H) for inserting at least part of a nozzle of an eye drop dispenser; and
- a sight orifice (A3) formed through one of the walls of the housing (H) in a position adjacent to said inlet opening (A1),
**characterized in that**:
- said first inlet opening (A1) is associated with a first coupling element configured for coupling a nozzle of a first dispenser type and providing a first droplet fall trajectory for the eye drop applied by said first dispenser type towards the user's eye;
- the housing (H) comprises a second inlet opening (A2) formed through one of the walls of the housing (H) in a position adjacent to said sight orifice (A3), said second inlet opening (A2) being associated with a second coupling element configured for coupling a nozzle of a second dispenser type and providing a second droplet fall trajectory for the eye drop applied by said second dispenser type towards the user's eye; and
- said sight orifice (A3) is located between said first and second inlet openings (A1, A2) in a position such that a sight line of the user's eye on which the housing (H) is arranged through the sight orifice (A3) does not cross either of said first and second droplet fall trajectories or their prolongations.

2. The applicator device according to claim 1, **characterized in that** the first eye drop dispenser type is a multi-dose dispenser and the second eye drop dispenser type is a single-dose dispenser.

3. The applicator device according to claim 2, **characterized in that** the first coupling element comprises an inner screw thread (F) conjugated with a corresponding outer screw thread associated with said nozzle of the first eye drop dispenser type.

4. The applicator device according to claim 3, **characterized in that** said inner screw thread (F) is formed in at least part of an inner contour of a first tubular portion (C1) communicating with the first inlet opening (A1) or including same as part of the inner opening thereof.

5. The applicator device according to claim 2, 3 or 4, **characterized in that** the second coupling element comprises a conical seat (C2i) having a section increasing from the second inlet opening (A2) to the outside of the housing (H), said conical seat (C2i) being conjugated with an outer conical surface associated with said nozzle of the second eye drop dispenser type.

6. The applicator device according to claim 5, **characterized in that** said conical seat (C2i) is formed in at least part of an inner contour of a second tubular portion (C2) which projects outwardly and/or inwardly with respect to the housing (H) from at least one of the walls thereof, and communicating with the second inlet opening (A2) or including same as part of the inner opening thereof.

7. The applicator device according to claim 6, **characterized in that** the second coupling element comprises two grooves (R1, R2) defmed in opposite locations of the conical seat (C2i).

8. The applicator device according to claim 4 or 6, **characterized in that** the first and second coupling elements are coaxial with respective first and second central geometric axes (E1, E2) substantially parallel to one another, and said sight orifice (3) is coaxial with a third central axis (E3) forming an angle between 30 and 45 degrees with respect to said first and second central geometric axes (E1, E2).

9. The applicator device according to claim 8, **characterized in that** when the housing (H) is arranged in a position of use on the facial area surrounding the user's eye socket and the user's head is in a tilted-back position, the first and second central geometric axes (E1, E2) are in substantially vertical positions.

10. The applicator device according to any one of the preceding claims, **characterized in that** said lower perimetral edge of the walls of the housing (H) comprises at least one closing portion (D1) adopting a curvature suitable for fitting with a portion of the user's face adjacent to the eye when in use.

11. The applicator device according to claim 10, **characterized in that** said lower perimetral edge of the walls of the housing (H) comprises an occlusion portion (D2) adopting a curvature suitable for applying pressure on the lacrimal sac of the user's eye when in use.

12. The applicator device according to any one of the preceding claims, **characterized in that** said walls of the housing (H) comprise a top wall (Sh) and side walls (Lh), and the first inlet opening (A1) is defined partially in said top wall (Sh) of the housing (H) and partially **in that** side wall (Lh) which is adjacent to the upper eyelid of the user's eye in a position of use.

13. The applicator device according to any one of the preceding claims, **characterized in that** said walls of the housing (H) comprise a top wall (Sh) and side walls (Lh), and the second inlet opening (A2) is defined in said top wall (Sh) of the housing (H) with a certain inclination with respect to a plane forming at least one region of the top wall (Sh) surrounding the second inlet opening (A2).

14. The applicator device according to any one of the preceding claims, **characterized in that** said walls of the housing (H) comprise a top wall (Sh) and side walls (Lh), and the sight orifice (A3) is defined in a plane forming at least one region of said top wall (Sh) surrounding the sight orifice (A3).

15. The applicator device according to any one of the preceding claims, **characterized in that** the housing (H) is made up of a one-piece body integrally incorporating the first and second coupling elements.

## Amended claims

### Amended claims in accordance with Rule 137(2) EPC.

**1.** An eye drop applicator device of the type comprising:
- a housing (H) having walls defining an open-bottom shell and having a lower perimeter edge configured for being adapted to the facial area surrounding a user's eye socket;
- a first inlet opening (A1) formed through one of said walls of the housing (H) for inserting at least part of a nozzle of an eye drop dispenser; and
- a sight orifice (A3) formed through one of the walls of the housing (H) in a position adjacent to said inlet opening (A1),
**characterized in that**:
- said first inlet opening (A1) is associated with a first coupling element configured for coupling a nozzle of an eye drop dispenser of a first type and providing a first droplet fall trajectory for the eye drop applied by said eye drop dispenser of a first type towards the user's eye;
- the housing (H) comprises a second inlet opening (A2) formed through one of the walls of the housing (H) in a position adjacent to said sight orifice (A3), said second inlet opening (A2) being associated with a second coupling element configured for coupling a nozzle of an eye drop dispenser of a second type, wherein said nozzle of said eye drop dispenser of a second type is different to said nozzle of said eye drop dispenser of a first type, and providing a second droplet fall trajectory for the eye drop applied by said eye drop dispenser of a second type towards the user's eye; and
- said sight orifice (A3) is located between said first and second inlet openings (A1, A2) in a position such that a sight line of the user's eye on which the housing (H) is arranged through the sight orifice (A3) does not cross with any of said first and second droplet fall trajectories nor with their prolongations.

**2.** The applicator device according to claim 1, **characterized in that** the first coupling element is configured for coupling a nozzle of an eye drop dispenser of said first type which is a multi-dose dispenser and the second coupling element is configured for coupling a nozzle of an eye drop dispenser of said second type which is a single-dose dispenser.

**3.** The applicator device according to claim 2, **characterized in that** the first coupling element comprises an inner screw thread (F) conjugated with a corresponding outer screw thread associated with said nozzle of the eye drop dispenser of said first type.

**4.** The applicator device according to claim 3, **characterized in that** said inner screw thread (F) is formed in at least part of an inner contour of a first tubular portion (C1) communicating with the first inlet opening (A1) or including same as part of the inner opening thereof.

**5.** The applicator device according to claim 2, 3 or 4, **characterized in that** the second coupling element comprises a conical seat (C2i) having a section increasing from the second inlet opening (A2) to the outside of the housing (H), said conical seat (C2i) being conjugated with an outer conical surface associated with said nozzle of the eye drop dispenser of said second type.

**6.** The applicator device according to claim 5, **characterized in that** said conical seat (C2i) is formed in at least part of an inner contour of a second tubular portion (C2) which projects outwardly and/or inwardly with respect to the housing (H) from at least one of the walls thereof, and communicating with the second inlet opening (A2) or including same as part of the inner opening thereof.

**7.** The applicator device according to claim 6, **characterized in that** the second coupling element comprises two grooves (R1, R2) defined in opposite locations of the conical seat (C2i).

**8.** The applicator device according to claim 4 or 6, **characterized in that** the first and second coupling elements are coaxial with respective first and second central geometric axes (E1, E2) substantially parallel to one another, and said sight orifice (3) is coaxial with a third central axis (E3) forming an angle between 30 and 45 degrees with respect to said first and second central geometric axes (E1, E2).

**9.** The applicator device according to claim 8, **characterized in that** the housing (H) and the first and second inlet openings (A1, A2) are configured such that the first and second central geometric axes (E1, E2) are in substantially vertical positions when the housing (H) is arranged in a position of use on the facial area surrounding the user's eye socket and the user's head is in a tilted-back position.

**10.** The applicator device according to any one of the preceding claims, **characterized in that** said lower perimeter edge of the walls of the housing (H) comprises at least one closing portion (D1) adopting a curvature complementary to a portion of the user's face adjacent to the eye.

**11.** The applicator device according to claim 10, **characterized in that** said lower perimeter edge of the walls of the housing (H) comprises an occlusion portion (D2) adopting a convex curvature, wherein said occlusion portion (D2) is located in an area of said lower perimeter edge staying, when in use, over the lacrimal sac of the user's eye, for pressing thereon.

**12.** The applicator device according to any one of the preceding claims, **characterized in that** said walls of the housing (H) comprise a top wall (Sh) and side walls (Lh), and the first inlet opening (A1) is defined partially in said top wall (Sh) of the housing (H) and partially **in that** side wall (Lh) which is adjacent to the upper eyelid of the user's eye in a position of use.

**13.** The applicator device according to any one of the preceding claims, **characterized in that** said walls of the housing (H) comprise a top wall (Sh) and side walls (Lh), and the second inlet opening (A2) is defined in said top wall (Sh) of the housing (H) inclined with respect to a plane forming at least one region of the top wall (Sh) surrounding the second inlet opening (A2).

**14.** The applicator device according to any one of the preceding claims, **characterized in that** said walls of the housing (H) comprise a top wall (Sh) and side walls (Lh), and the sight orifice (A3) is defined in a plane forming at least one region of said top wall (Sh) surrounding the sight orifice (A3).

**15.** The applicator device according to any one of the preceding claims, **characterized in that** the housing (H) is made up of a one-piece body integrally incorporating the first and second coupling elements.
